# EUROPEAN PATENT SPECIFICATION

(11) **EP 1 355 596 B1**
(45) Date of publication and mention of the grant of the patent: **16.12.2009**
(21) Application number: 02705883.3
(22) Date of filing: 23.01.2002
(51) Int. Cl.: A61F 5/00

(54) **JOINT BRACE WITH MULTI-PLANAR PIVOTING ASSEMBLY**
GELENKSCHIENE MIT MEHREBENEN-SCHWENKANORDNUNG
DISPOSITIF DE MAINTIEN D'ARTICULATION PRESENTANT UN SYSTEME DE PIVOTEMENT MULTI-PLANAIRE

(30) Priority: 29.01.2001 US 771763; 02.02.2001 US 776523
(43) Date of publication of application: 29.10.2003
(73) Proprietor: Asterisk.Asterisk, LLC, Foothill Ranch, CA 92610 (US)
(72) Inventor: FREEMAN, Brad, Mission Viejo, CA 92691 (US); CASTILLO, James, Los Alamos, CA 93440 (US)
(74) Representative: Mackenzie, Andrew Bryan
(86) International application number: PCT/US2002/001759
(87) International publication number: WO 2002/060359

(56) References cited:
- DE-A1- 1 491 569
- US-A- 3 901 223
- US-A- 5 063 916
- US-A- 5 267 946
- US-A- 5 277 698
- US-A- 5 547 464
- US-A- 5 571 206

## Description

### BACKGROUND OF THE INVENTION

This invention relates in general to braces for joint support, and in particular to an exteriorly positionable anatomical brace having a pivoting joint assembly with multi planar hinging for automatic alignment of joined limb structures in relation to each other, and additionally having an infinitely-adjustable, cable-controlled limb extension regulator.

The features of the preamble of claim 1 are known from the document US-A-5 277 698.

Both injury and disease can affect the health, well-being, and operability of various joints of the human body. Chief among such joints are the knee and elbow where disease such as osteo-arthritis can curtail normal activity or where an injury such as a sports-related abuse or impact can prevent or severely limit continued activity. One manner of treating such joint conditions is to fit the wearer with an appropriate brace whereby a pivotal support member is positioned adjacent the affected joint and held in place usually by cuffs situated around limb structure sites above and below the supported joint. As is apparent, the cuffs are responsible for stabilizing the support member and therefore must be well secured to their associated limbs. In addition to requiring proper limb structure embrace by cuffs, a joint brace also requires a joint pivoting assembly that supports, stabilizes, and protects the actual joint itself while pivotally joining the cuffs. Thus, in the knee joint for example, the joint pivoting assembly of the brace most beneficially should pivot in one bending or extension plane while also permitting multi planar motion such that the lower leg beneath the knee can be moved in a normal manner and the upper and lower leg structures can align with each other in a natural manner. Further, it many times is desirable to be able to precisely and infinitely limit or regulate the distance of the pivotal extension plane at the knee while allowing natural bendability and normal multi planar motion up to the controlled extension distance. Unfortunately, however, present braces generally are notable to offer multi-planar alignment capabilities or infinite extension control, thereby requiring a user to endure single-plane pivotability along with either a self limit or pre-set limit of limb extendability. In view of such restrictions, it is apparent that a need is present for a joint brace that permits substantially natural limb movement in conjunction with limb extension control as indicated for particular limb care.

Consequently, a primary object of the present invention is to provide a joint brace having a joint assembly with multi planar hinging for automatic alignment of joined limb structures in relation to each other.

Another object of the present invention is to provide a joint brace having an infinitely-adjustable limb extension regulator for limiting limb extension as indicated for a particular user.

These and other objects of the present invention will become apparent throughout the description thereof which now follows

### BRIEF SUMMARY OF THE INVENTION

The present invention is an exteriorly positionable anatomical brace as recited in Claim 1. The brace is for stabilizing a uniting pivoting joint such as a knee joint disposed between a first and second limb structure of a living being. The brace comprises an upper frame member and a lower frame member joined together by a pivoting joint member, with each such frame member having secured thereon a respective cuff for encompassing a portion of each limb structure above and below the joint. Retention of the brace in place at the joint site is preferably accomplished with respective upper and lower securement members each wrapping around a respective limb structure in alignment with and not encompassed by the cuff The pivoting joint member comprises two opposing pivoting assemblies each positionable on one side of the anatomical joint of a wearer to thus join the upper and lower frame members together. Each of these assemblies includes a forward arm member and a rearward arm member each having an upper end and a lower end, with these ends connected respectively to the upper frame member and the lower frame member. Specifically, the upper ends of each arm are individually mounted within a spherically-pivotal socket in connection with the upper frame member, while the lower ends of each arm likewise are individually mounted within a spherically-pivotal socket in connection with the lower frame member. As is apparent, these individual spherical mounts permit the selection of differing pivot ratios at a total of eight sites (four sites per lateral and per medial side) to thereby enable the upper and lower frame members to assume many different angular relationships with each other. Because of the availability of such a vast number of relationship combinations, the frame members of the brace becomes substantially self-aligning with each individual joint encounter among many wearers, thus accomplishing simulation of actual limb movement and angular interrelations thereof as natural individual limb-structure correlations are maintained.

As earlier noted, proper joint care many times requires limited or regulated limb extension, with such control emanating at the pivoting joint member. While prior art controls typically include inserts of a predetermined size for placement in the base path of upper and lower frame travel, the limb extension regulator of the present invention is a cable, preferably fabricated of braided metal strands, extending between each rearward arm member and the upper frame member. A cable-length adjuster, preferably externally accessible, is provided for infinitely adjusting the length of cable available between the arm member and frame member to thereby regulate extendability of the brace-bearing limb. Most preferably, a visible measurement scale is provided for each cable such that available cable length on each side of the joint is adjusted to be substantially identical. In addition to being infinitely length-adjustable, the cable additionally provides a modicum of elasticity such that cessation of limb travel produces a less dramatic limb impact, but, instead, a gentler limb-extension termination for the wearer. The brace here defined therefore substantially simulates natural joint behavior along with extension control as individually indicated.

### BRIEF DESCRIPTION OF THE DRAWINGS

An illustrative and presently preferred embodiment of the invention is shown in the accompanying drawings in which:
Figure 1 is a perspective lateral view of a knee brace with upper and lower cuffs of respective upper and lower frame members in place on a patient leg shown in phantom;
Figure 2 is a perspective medial view of the knee brace of Figure 1;
Figure 3 is a lateral perspective view of the upper cuff and upper frame member only of Figure 1 in disassociated relationship;
Figure 4 is a medial perspective view of the upper cuff and upper frame member only of Figure 3;
Figure 5 is a rear perspective view of the upper cuff and upper frame member of Figure 1 in place on a leg;
Figure 6 is an interior perspective view of a portion of the upper cuff of Figure 1;
Figure 7a is an interior side elevation view of the upper cuff of Figure 4;
Figure 7b is a schematic interior side elevation view of the cuff of Figure 7a showing tensioning thereof;
Figure 7c is a top plan view along line 7c-7c of Figure 7a;
Figure 8 is an inner perspective view of the joint assembly and respective portions of joined upper and lower frame members of Figure 1;
Figure 9 is an exploded perspective view of the joint assembly and frame members of Figure 8;
Figures 10a and 10b are perspective views of the inner and outer sides of the joint assembly of Figure 8; and
Figure 11 is an exploded perspective view of the joint assembly of Figure 10a.

### DETAILED DESCRIPTION OF THE INVENTION

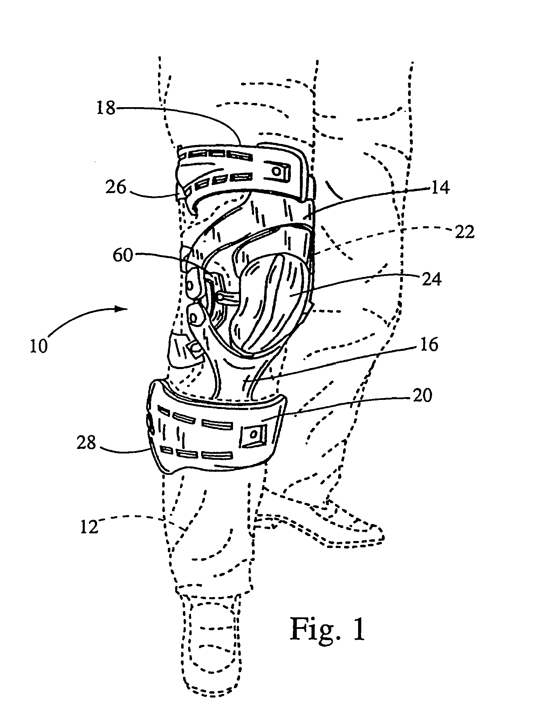

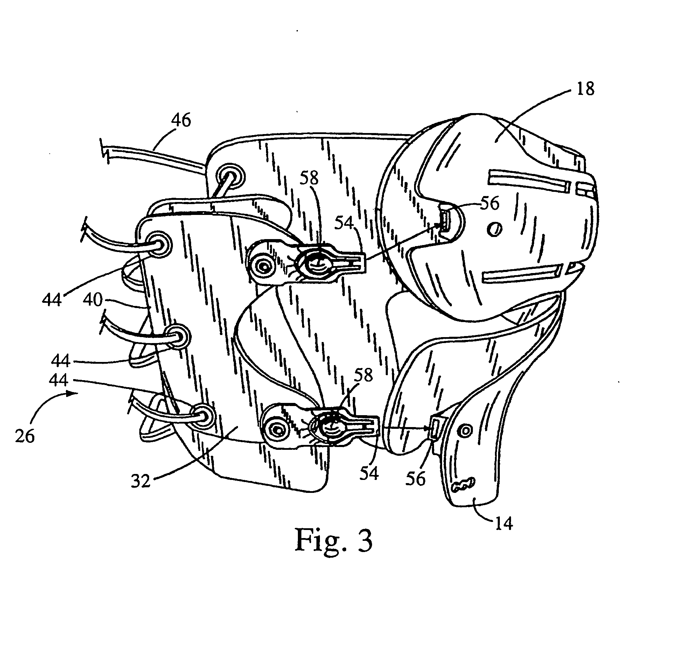

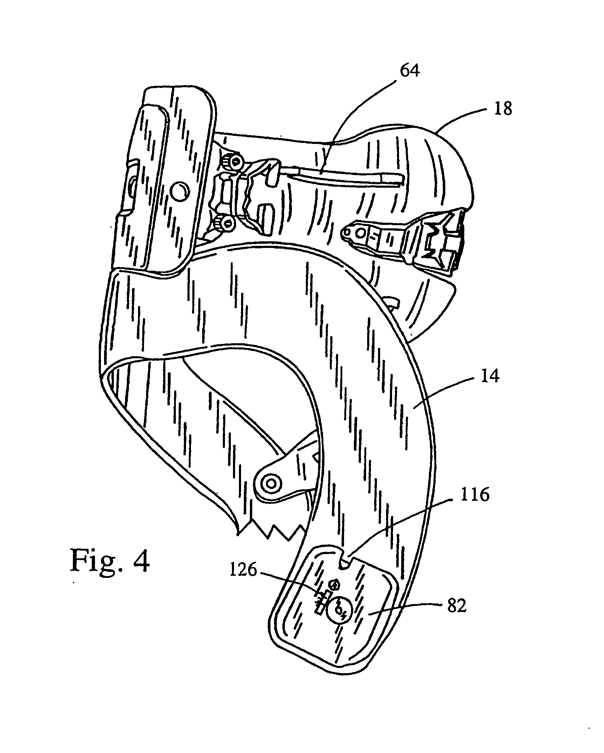

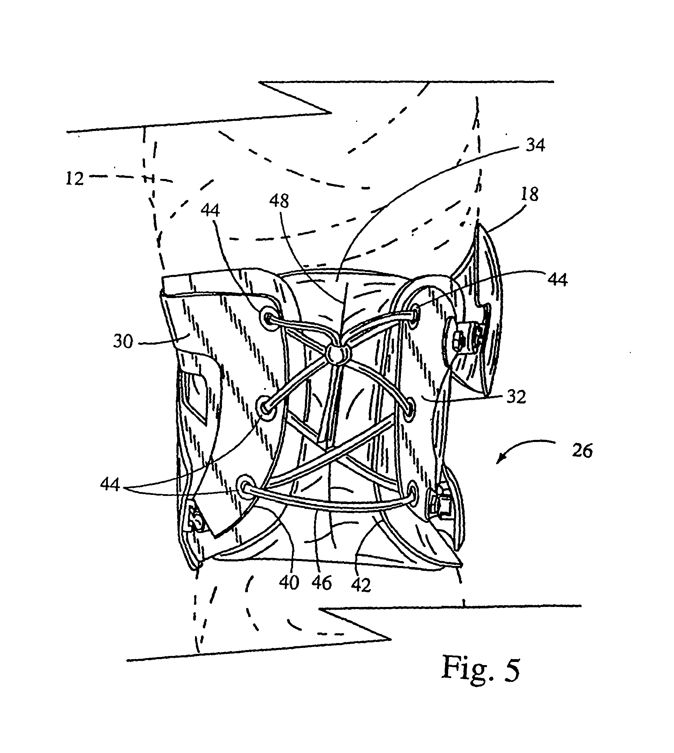

Referring first to Figures 1-5, a knee brace 10 is shown (Figure 1) in place on a leg 12 of a human being. The brace 10 has an upper frame member 14 and a lower frame member 16, with each such frame member 14, 16 having secured thereon a respective cuff 18, 20 for disposition about the limb structures above and below the knee joint 22. Each cuff 18, 20 is an arcuate wall structure, which non-limitedly can be fabricated of a polymer plastic, for juxtapositioning with the respective limb structures as shown. A snap-in protective patella cup 24 can be included as shown for specific impact absorption that may occur at the patella of the knee joint 22.

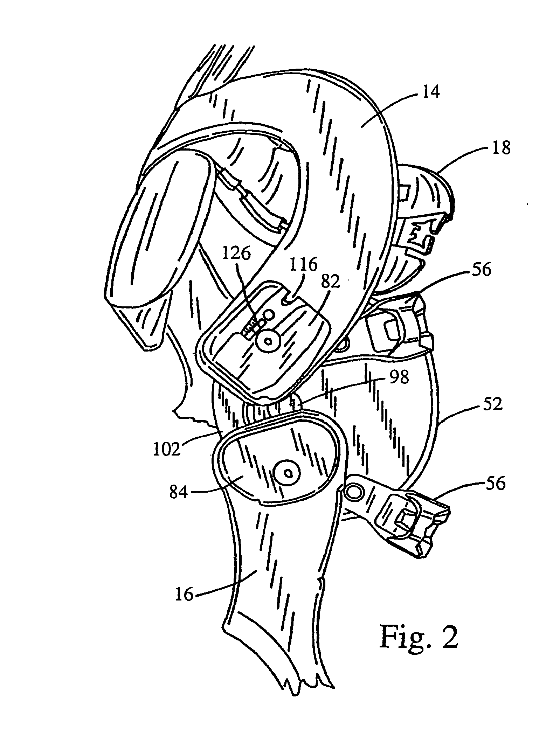

The knee brace 10 is retained in place on the leg 12 with respective upper and lower securement members 26, 28 each respectively wrapping around an adjacent rear portion of the leg 12. While Figures 2-5 show only the upper securement member 26, it is to be understood that the following description thereof applies equally to the lower securement member 28. Thus, the securement member 26 includes a medial piece 30 and a lateral piece 32 each attached at outside edges thereof to an elastomeric central piece 34 disposed behind the medial and lateral pieces 30, 32. Respective inside edges 40, 42 of the medial and lateral pieces 30, 32 are provided with eyelets 44 through which is intertwined a length of non-elastomeric lace 46 in substantially the same manner as a shoe is laced to thereby permit the drawing of each inside edge 40, 42 toward each other. As would be recognized by the skilled artisan, hook-and-loop connectors (e.g. VELCRO) or other appropriate engagers can be employed in place of the length of lace 46. Finally, the elastomeric central piece 34 is secured along a generally central vertical length 48 thereof to a liner section (not shown) situated behind the central piece 34 to thereby permit elasticized movement of the medial and lateral pieces 30, 32.

The lateral piece 32 is releasably secured respectively to the upper cuff 18 and the upper frame member 14, and the medial piece 30 is releasably secured to the upper frame member 14 and the medial condyle 52, all by way of respective quick-release tab members 54 situated within respective slots 56. As shown, each tab member 54 is provided with a finger-receiving pressure button 58 which, when depressed, permits removal of the tab member 54 from the slot 56. In operation, the brace 10 is placed at the limb site of a user and positioned about the involved limb structures. Upon first placement of the brace 10, the lace 46 is tightened to appropriate tightness while the central piece 34 increases surface area on the leg 12 to disperse pressure and prevent pull from the leg 12 such that the cuff 18 is properly maintained in place. Once such lacing is accomplished the first time, re-lacing is not required during brace use. Specifically, when a user wishes to remove the brace, the user simply presses inwardly on the pressure buttons 58 of only laterally, or, preferably, only medially, situated tab members 54 to release these tab members 54 from their respective slots 56 and remove the brace 10 from the leg 12. It is important to note that the above-described tab-member release does not require increased tension on the leg and therefore is both safe and comfortable. Subsequent re-positioning of the brace 10 merely requires placement thereof as previously situated and re-connection of the earlier disengaged tab members 54 into respective slots 56. This re-connection requires no contact with, or re-adjustment of, the lace 46 or the central piece 34, and thereby assures proper brace placement without awkward, and very possibly incorrect, orientation of the brace 10. Because the medial connection involves connection to the medial condyle 52 which is, of course, at the hinge point of the upper and lower frame members 14, 16, a closer positioning of the securement member 26 to the body joint is permitted, thereby improving joint support. While a lateral condyle 60 does not bear a connector member, it is to be understood that such construction could be provided if desired.

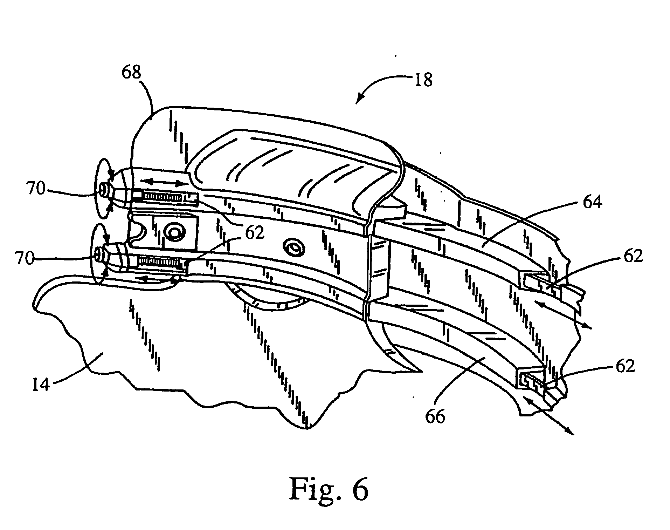

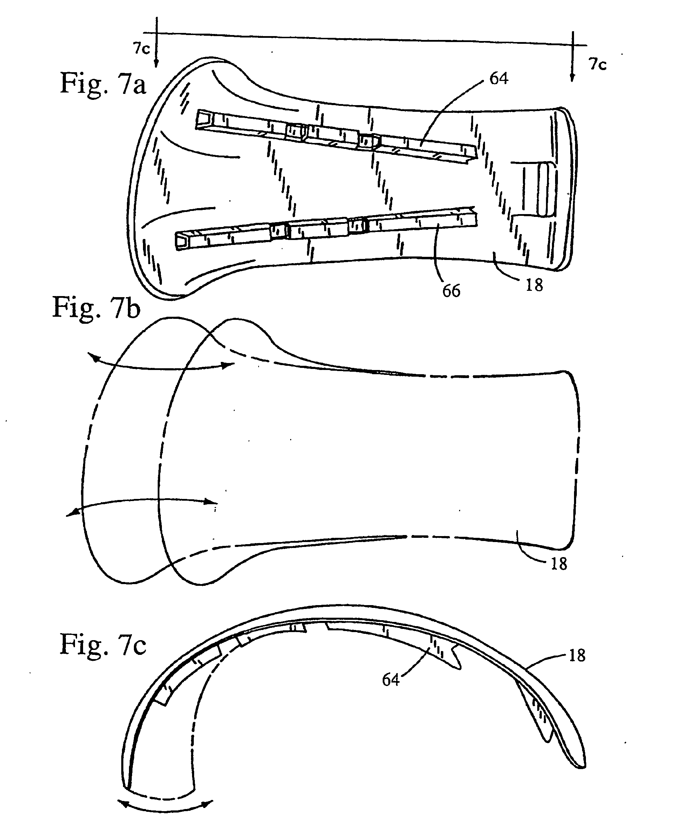

Construction of the cuffs 18, 20 is illustrated in Figures 6-7c. Both the upper cuff 18 and lower cuff 20 are substantially identical in construction except for overall size since, of course, the lower cuff 20 encompasses a smaller-diameter limb portion below the knee joint 22. As shown particularly in Figures 6 and 7a with respect to the upper cuff 18, whose following description also applies to the lower cuff 20, the cuff 18 has two tensioning strip members 62, integral therewith and disposed within respective noncontinuous sleeves 64, 66 that are structurally a part of the cuff 18 and that converge toward each other medially. Each strip member 62, which preferably is fabricated of titanium, stainless steel, or similar material possessing similar tensioning properties, continues medially into a cuff mount 68 that functions to secure the cuff 18 to the upper frame member 14. Finally, a respective exteriorly-accessible threaded screw 70 extends into each strip member 62 for adjusting tension in each strip member 62 and simultaneously adjusting the arc defined by the upper cuff 18. Thus, clockwise turning of the screw 70 incrementally draws the lateral end of the strip member 62 medially for arcuately tightening the cuff 18, while counter clockwise turning of the screw 70 incrementally releases the lateral end of the strip member 62 for arcuately loosening the cuff 18. Operationally, the brace 10 is fitted to a patient by encompassing the cuffs about the respective limb structures above and below the knee joint 22 as seen in Figure 1. Once the upper cuff 18 is situated about the limb structure, the screws 70 are threadingly advanced to thereby cause movement of the lateral end of the cuff 18, as illustrated in Figures 7b and 7c, against the limb structure as the strip members 62 are forced to bend toward the encompassed limb structure. Continued screw advancement increases tightening of the cuff 18 against the encompassed limb structure to thereby accomplish superior anchoring of the brace 10 and consequent stabilization of the knee joint 22. As earlier noted, the lower cuff 20 is constructed in the same manner as the upper cuff 18 and therefore encompasses and embraces the limb structure below the knee joint 22 in like fashion.

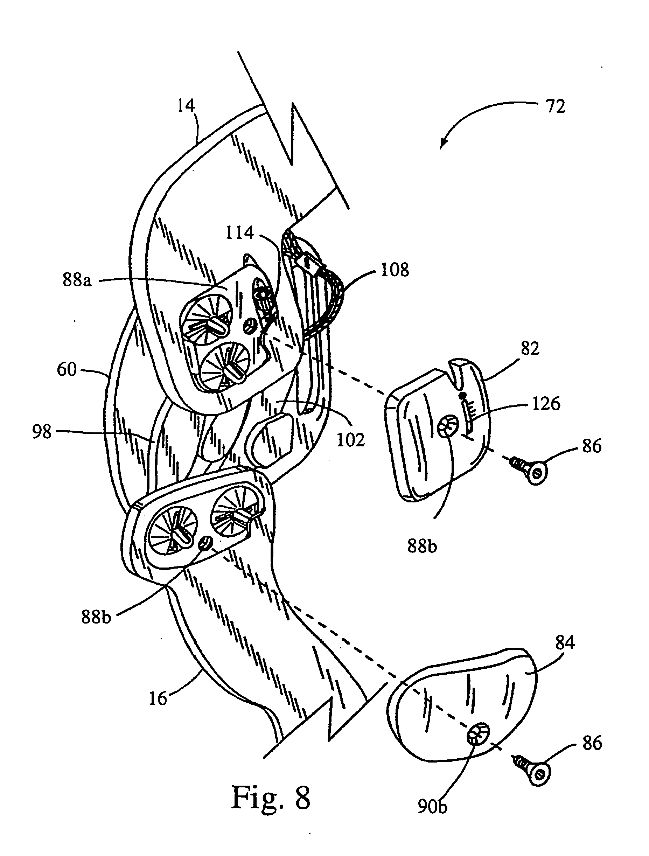

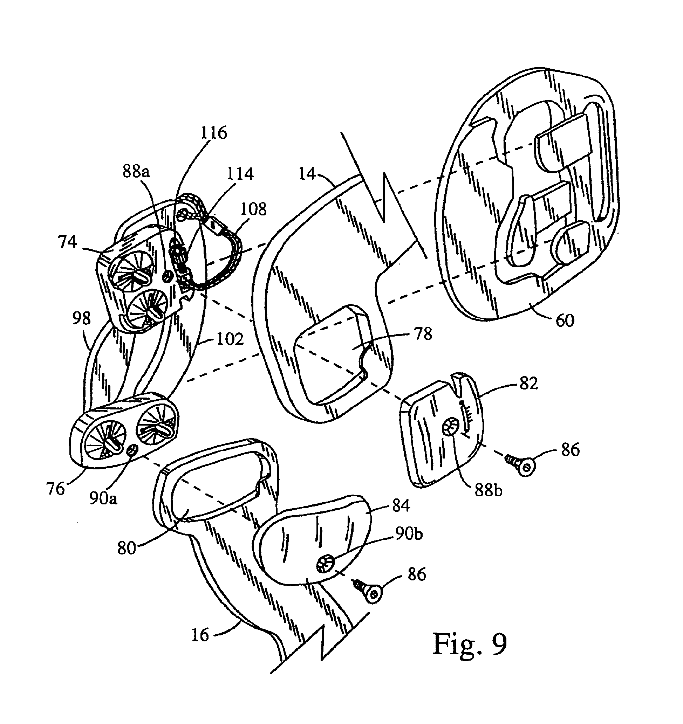

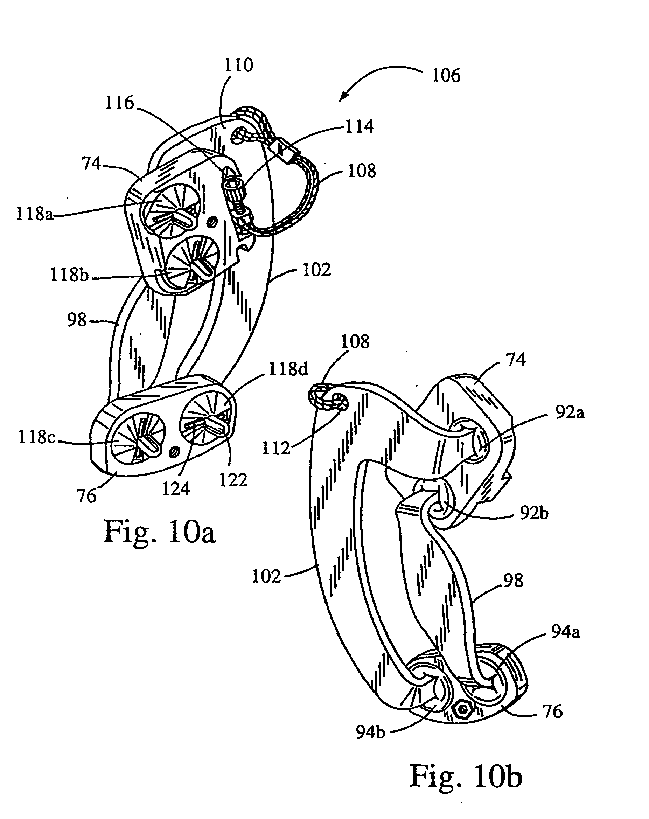

Referring to Figures 8-11, the pivoting assembly 72 uniting the upper and lower frame members 14,16 is illustrated. The assembly 72 includes an upper housing 74 and a lower housing 76 that fit, respectively, into a complementarity shaped opening 78 of the upper frame member 14 and a complementarity shaped opening 80 of the lower frame member 16. Once so positioned, respective caps 82, 84 are held in place with conventional set screws 86 passing respectively through apertures 88a, 88b and 90a, 90b. Those skilled in the art however will recognize that the housings 74 and 76 can be formed unitary with the frame members 14 and 16. The lateral condyle 60 resides between the assembly 72 and the knee joint 22. Both the upper and lower housings 74, 76 have two respective openings 92a, 92b and 94a, 94b each having respective sidewalls 96 shaped to nest a spherical shape. Disposed between two openings 92b, 94a of the housings 74, 76 is a forward arm member 98 having generally perpendicularly angled first and second ends 100a, 100b directable toward the openings 92b, 94a. In like manner, a rearward arm member 102 having generally perpendicularly angled first and second ends 104a, 104b is disposed between two openings 92a, 94b of the housings 74, 76 such that the ends 104a,104b are directable toward the openings 92a, 94b. A cable assembly 106 includes a cable 108 extending from the upper housing 74 to an upper edge portion 110 through an aperture 112 of the rearward arm member 102, and is provided with a conventional set screw 114 at one end thereof for extending or shortening the length of the cable 108 disposed between the rearward arm member 102 and upper housing 74. Such length adjustment is accomplished with an Allen wrench inserted into the enterable channel 116 leading to the set screw 114. Because the upper housing 74 resides within the upper frame member 14, the cable 108 functions as a joint extension limiter to determine the travel distance of the upper frame member 14 from the joint and thus the pivotal distance of the upper and lower frame members 14, 16 in relation to each other. An opening 126 can be provided in the cap 82 such that the progressive placement of the cable 108 can be observed exteriorly and such placement can be made identical for both the lateral and medial sides. Two additional benefits are provided by the cable 108 in that, first, infinite pivot-distance adjustability, as opposed to prior-art pre-sized stop members, allows great flexibility in leg extension, and, second, the cable itself has a dampening, or minimal stretch, effect that results in a softer extension stop and a consequent reduced risk of joint trauma.

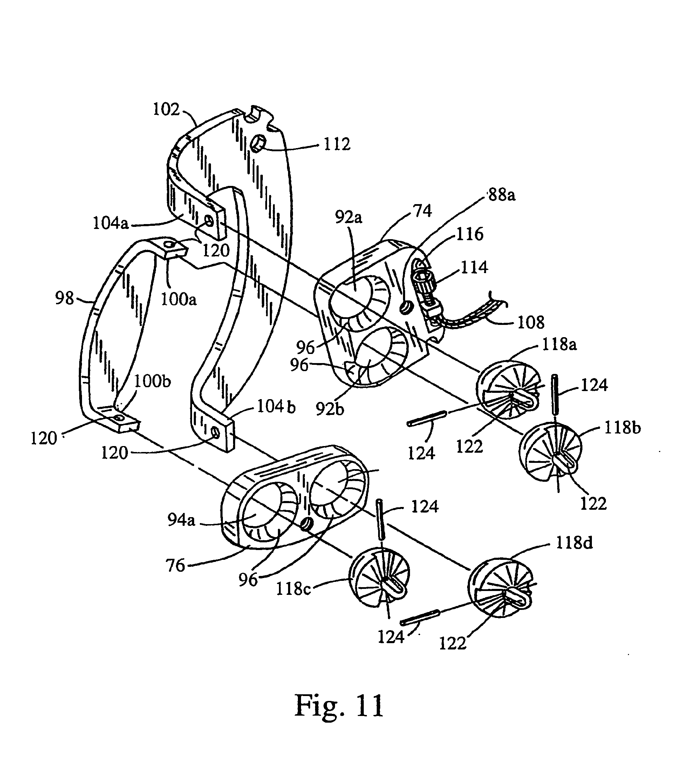

As earlier described, the sidewalls 96 of the openings 92a, 92b and 94a, 94b are shaped to nest spherical forms. As clearly illustrated in Figure 11, spherical sockets 118a,118b,118c,118d are disposed in these openings 92a, 92b and 94a, 94b in the constructed assembly 72, and each such socket accepts one respective perpendicularly angled end of forward and rearward arm members 98,102. Each angled end 100a, 100b, 104a,104b has an aperture 120 there through which mates with a transverse aperture 122 of each socket 118a, 118b, 118c, 118d such that respective pins 124 can pass through such mated apertures and retain the angled ends 100a, 100b, 104a, 104b within the sockets 118a, 118b, 118c, 118d. Because of the spherical interface between each socket 118a, 118b, 118c, 118d and each sidewall 96, multi planar movement of the upper and lower frame members 14,16 in relation to each other can be accomplished. In particular, the different pivot points thus provided allow different pivot ratios as needed for both lateral and medial sides to thereby simulate actual knee joint movement. This is, of course, in contrast to parallel planar hinges as found in the prior art where the knee joint and limb structures of a user are forced to adapt to knee brace-construction instead of the knee brace adapting to the needs of the user. The present knee brace 10, because of the multi planar and potentially differing pivot ratios and consequent multi planar movement capabilities of the lower frame member 16 in relation to the upper frame member 14, provides automatic tibia alignment and automatic anatomical changes over time by accommodating anatomical differences among users. These properties accomplish all-important positive three-point positioning at the quadriceps muscle, the gastrocnemius (calf) muscle, and the knee joint itself. In this manner, stabilization and support of a uniting pivoting joint occurs economically, through an "off the-shelf' brace, and, simultaneously, most effectively through continual self-alignment capabilities combined with sound limb-structure stability.

While an illustrative and presently preferred embodiment of the invention has been described in detail herein, it is to be understood that the inventive concepts may be otherwise variously embodied and employed and that the scope of protection is defined by the appended claims

## Claims

1. An exteriorly positionable anatomical brace (10) for stabilizing a uniting pivoting joint disposed between a first limb structure and a second limb structure of a living being, the brace (10) comprising:
a) an upper frame member (14) and a lower frame member (16);
b) an upper cuff (18) for encompassing a portion of the first limb structure and secured to the upper frame member (14), and a lower cuff (20) for encompassing a portion of the second limb structure and secured to the lower frame member (16);
c) an upper securement member (26) for securing the upper cuff (18) to the first limb structure, and a lower securement member (28) for securing the lower cuff (20) to the second limb structure; and
d) a pivoting joint member (72) connecting the upper (14) and lower (16) frame members, said joint member (72) comprising two opposing pivoting assemblies each respectively positionable on one side of the uniting pivoting joint, **characterised by** each said pivoting assembly comprising:
i) two upper spherically-pivotal socket mounts disposed in the upper frame member (14);
ii) two lower spherically-pivotal socket mounts disposed in the lower frame member (16);
iii) a forward arm member (98) having an upper end being pivotally retained in a rearward one of the upper spherically-pivotal socket mounts and a lower end being pivotally retained in a forward one of the lower spherically-pivotal socket mounts; and
iv) a rearward arm member (102) having an upper end being pivotally retained in a forward one of the upper spherically-pivotal socket mounts and a lower end being pivotally retained in a rearward one of the lower spherically-pivotal socket mounts.

2. An exteriorly positionable anatomical brace (10) as claimed in Claim 1 wherein the two upper spherically-pivotal socket mounts have pivot ratios differing from each other.

3. An exteriorly positionable anatomical brace (10) as claimed in Claim 1 wherein the two lower spherically-pivotal socket mounts have pivot ratios differing from each other.

4. An exteriorly positionable anatomical brace (10) as claimed in Claim 1 wherein each of the spherically-pivotal socket mounts has a pivot ratio different from all other socket mounts.

5. An exteriorly positionable anatomical brace (10) as claimed in Claim 1 further comprising a substantially infinitely adjustable limb extension regulator (108) for demarcating limb extension range.

6. An exteriorly positionable anatomical brace (10) as claimed in Claim 5 wherein the two upper spherically-pivotal socket mounts have pivot ratios differing from each other.

7. An exteriorly positionable anatomical brace (10) as claimed in Claim 5 wherein the two lower spherically-pivotal socket mounts have pivot ratios differing from each other.

8. An exteriorly positionable anatomical brace (10) as claimed in Claim 5 wherein each of the spherically-pivotal socket mounts has a pivot ratio different from all other socket mounts.

9. An exteriorly positionable anatomical brace (10) as claimed in Claim 5 wherein the limb extension regulator is an expanse of a length of cable (108) extending between the rearward arm member (102) and the upper frame member (14).

10. An exteriorly positionable anatomical brace (10) as claimed in Claim 9 wherein the length of cable (108) is fabricated of braided metal strands

11. An exteriorly positionable anatomical brace (10) as claimed in Claim 9 additionally comprising an exteriorly accessible controller (114) for lengthening or shortening the length of cable (108) extending between the rearward arm member (102) and the upper frame member (14), thereby regulating limb extension distance.

12. An exteriorly positionable anatomical brace (10) as claimed in Claim 11 wherein the exteriorly accessible controller is a threadably-engaged screw shaft (116) distally attached to the length of cable, said shaft (116) being tool-accessible for rotation and resulting lengthening or shortening of the length of cable (108).

13. An exteriorly positionable anatomical brace (10) as claimed in Claim 11 additionally comprising an externally visible measurement scale (126) for the length of cable (108) such that respective lengths of cable (108) extending between the rearward arm member'(102) and the upper frame member (14) of each opposing pivoting assembly of the pivoting joint member (72) can be made equal.

## Patentansprüche

1. Eine von außen anbringbare anatomische Schiene (10) zum Stabilisieren eines Verbindungs-Drehgelenks zwischen einer ersten Körperglied-Struktur und einer zweiten Körperglied-Struktur eines Lebewesens, wobei die Schiene (10) Folgendes umfasst:
a) einen oberen Rahmenteil (14) und einen unteren Rahmenteil (16);
b) eine an dem oberen Rahmenteil (14) befestigte obere Manschette (18) zum Umgreifen eines Teils der ersten Körperglied-Struktur, und eine an dem unteren Rahmenteil (16) befestigte untere Manschette (20)zum Umgreifen eines Teils der zweiten Körperglied-Struktur;
c) einen oberen Befestigungsteil (26) zum Befestigen der oberen Manschette (18) an der ersten Körperglied-Struktur und einen unteren Befestigungsteil (28) zum Befestigen der unteren Manschette (20) an der zweiten Körperglied-Struktur; und
d) einen Schwenkgelenkteil (72), der die oberen (14) und unteren (16) Rahmenteile verbindet, wobei der Gelenkteil (72) zwei gegenüberliegende Schwenkbaugruppen umfasst, die jeweils auf einer Seite des Schwenkverbindungsgelenkes anzuordnen sind,
**dadurch gekennzeichnet, dass** die jede Schwenkbaugruppe Folgendes umfasst:
i) zwei obere kugelförmig verschwenkbare Buchsenbefestigungen, die in dem oberen Rahmenteil (14) angeordnet sind;
ii) zwei untere kugelförmig verschwenkbare Buchsenbefestigungen, die in dem unteren Rahmenteil (16) angeordnet sind;
iii) einen vorderen Armteil (98), der ein oberes Ende, das schwenkbar in einem hinteren einen der oberen kugelförmig verschwenkbaren Buchsenbefestigungen festgehalten ist und ein unteres Ende aufweist, das schwenkbar in einem vorderen einen der unteren kugelförmig verschwenkbaren Buchsenbefestigungen festgehalten ist; und
iv) einen hinteren Armteil (102), der ein oberes Ende, das schwenkbar in einem vorderen einen der oberen kugelförmig verschwenkbaren Buchsenbefestigungen gehalten ist, und ein unteres Ende aufweist, das schwenkbar in einem hinteren einen der unteren kugelförmig verschwenkbaren Buchsenbefestigungen festgehalten ist.

2. Eine von außen anbringbare anatomische Schiene (10) nach Anspruch 1, bei der die zwei oberen kugelförmig verschwenkbaren Buchsenbefestigungen Schwenkverhältnisse aufweisen, die voneinander verschieden sind.

3. Eine von außen anbringbare anatomische Schiene (10) nach Anspruch 1, bei der die zwei unteren kugelförmig verschwenkbaren Buchsenbefestigungen Schwenkverhältnisse aufweisen, die voneinander verschieden sind.

4. Eine von außen anbringbare anatomische Schiene (10) nach Anspruch 1, bei der jede der kugelförmig verschwenkbaren Buchsenbefestigungen ein Schwenkverhältnis aufweist, das von dem aller anderen Buchsenbefestigungen verschieden ist.

5. Eine von außen anbringbare anatomische Schiene (10) nach Anspruch 1, die weiterhin einen im Wesentlichen unbegrenzt einstellbaren Körperglied-Streckungs-Regler (108) zum Demarkieren eines Körperglied-Streckungsbereiches umfasst.

6. Eine von außen anbringbare anatomische Schiene (10) nach Anspruch 5, bei der die zwei oberen kugelförmig verschwenkbaren Buchsenbefestigungen Schwenkverhältnisse aufweisen, die voneinander verschieden sind.

7. Eine von außen anbringbare anatomische Schiene (10) nach Anspruch 5, bei der die zwei unteren kugelförmig verschwenkbaren Buchsenbefestigungen Schwenkverhältnisse aufweisen, die voneinander verschieden sind.

8. Eine von außen anbringbare anatomische Schiene (10) nach Anspruch 5, bei der jede der kugelförmig verschwenkbaren Buchsenbefestigungen ein Schwenkverhältnis aufweist, das von dem aller anderen Buchsenbefestigungen verschieden ist.

9. Eine von außen anbringbare anatomische Schiene (10) nach Anspruch 5, bei der der Körperglied-Streckungsregler der Bereich einer Länge eines Seils (108) ist, das sich zwischen dem hinteren Armteil (102) und dem oberen Rahmenteil (14) erstreckt.

10. Eine von außen anbringbare anatomische Schiene (10) nach Anspruch 9, bei der die Länge des Seils (108) aus geflochtenen Metallsträngen hergestellt ist.

11. Eine von außen anbringbare anatomische Schiene (10) nach Anspruch 9, die zusätzlich eine von außen zugängliche Steuereinrichtung (114) zum Verlängern oder Verkürzen der Länge des Seils (103) umfasst, das sich zwischen dem hinteren Armteil (102) und dem oberen Rahmenteil (14) erstreckt, wodurch die Körperglied-Streckungsentfernung geregelt wird.

12. Eine von außen anbringbare anatomische Schiene (10) nach Anspruch 11, bei der die von außen zugängliche Steuereinrichtung eine in Gewindeeingriff stehende Schraubenwelle (116) ist, die distal an der Länge des Seils angebracht ist, wobei die Welle (116) für ein Werkzeug für eine Drehung und eine sich daraus ergebende Verlängerung oder Verkürzung der Länge des Kabels (108) zugänglich ist.

13. Eine von außen anbringbare anatomische Schiene (10) nach Anspruch 11, die zusätzlich eine von außen sichtbare Messskala (126) für die Länge des Seils (108) derart umfasst, dass die jeweiligen Längen des Seils (108), das sich zwischen dem hinteren Armteil (102) und dem oberen Rahmenteil (14) jeder gegenüberliegenden Schwenkbaugruppe des Schwenkgelenkteils (72) erstreckt, gleich gemacht werden können.

## Revendications

1. Appareil anatomique (10) pouvant être positionné extérieurement pour stabiliser une articulation pivotante d'union disposée entre une première structure de membre et une seconde structure de membre d'un être vivant, l'appareil (10), comprenant :
a) un élément de châssis supérieur (14) et un élément de châssis inférieur (16) ;
b) une manchette supérieure (18) pour englober une partie de la première structure de membre et fixée sur l'élément de châssis supérieur (14) et une manchette inférieure (20) pour englober une partie de la seconde structure de membre et fixée sur l'élément de châssis inférieur (16) ;
c) un élément de fixation supérieur (26) pour fixer la manchette supérieure (18) à la première structure de membre, et un élément de fixation inférieur (28) pour fixer la manchette inférieure (20) sur la seconde structure de membre ; et
d) un élément d'articulation pivotant (72) raccordant les éléments de châssis supérieur (14) et inférieur (16), ledit élément d'articulation (72) comprenant deux ensembles pivotants opposés chacun respectivement positionnable sur un côté de l'articulation pivotante d'union, **caractérisé en ce que** chaque dit ensemble pivotant comprend :
i) deux montants d'emboîture supérieurs sphériquement pivotants disposés dans l'élément de châssis supérieur (14) ;
ii) deux montants d'emboîture inférieurs sphériquement pivotants disposés dans l'élément de châssis inférieur (16) ;
iii) un élément de bras avant (98) ayant une extrémité supérieure qui est retenue de manière pivotante dans un montant arrière des montants d'emboîture supérieurs sphériquement pivotants et une extrémité inférieure qui est retenue de manière pivotante dans un montant avant des montants d'emboîture inférieurs sphériquement pivotants ; et
iv) un élément de bras arrière (102) ayant une extrémité supérieure qui est retenue de manière pivotante dans un montant avant des montants d'emboîture supérieurs sphériquement pivotants et une extrémité inférieure qui est retenue de manière pivotante dans un montant arrière des montants d'emboîture inférieurs sphériquement pivotants.

2. Appareil anatomique (10) pouvant être positionné extérieurement selon la revendication 1, dans lequel les deux montants d'emboîture supérieurs sphériquement pivotants ont des rapports de pivot qui diffèrent l'un de l'autre.

3. Appareil anatomique (10) pouvant être positionné extérieurement selon la revendication 1, dans lequel les deux montants d'emboîture inférieurs sphériquement pivotants ont des rapports de pivot qui diffèrent l'un de l'autre.

4. Appareil anatomique (10) pouvant être positionné extérieurement selon la revendication 1, dans lequel chacun des montants d'emboîture sphériquement pivotants a un rapport de pivot différent de tous les autres montants d'emboîture.

5. Appareil anatomique (10) pouvant être positionné extérieurement selon la revendication 1, comprenant en outre un régulateur d'extension de membre réglable sensiblement infiniment (108) pour délimiter la plage d'extension de membre.

6. Appareil anatomique (10) pouvant être positionné extérieurement selon la revendication 5, dans lequel les deux montants d'emboîture supérieurs sphériquement pivotants ont des rapports de pivot qui diffèrent l'un de l'autre.

7. Appareil anatomique (10) pouvant être positionné extérieurement selon la revendication 5, dans lequel les deux montants d'emboîture inférieurs sphériquement pivotants ont des rapports de pivot qui diffèrent l'un de l'autre.

8. Appareil anatomique (10) pouvant être positionné extérieurement selon la revendication 5, dans lequel chacun des montants d'emboîture sphériquement pivotants a un rapport de pivot différent de tous les autres montants d'emboîture.

9. Appareil anatomique (10) pouvant être positionné extérieurement selon la revendication 5, dans lequel le régulateur d'extension de membre est un prolongement d'une longueur de câble (108) s'étendant entre l'élément de bras arrière (102) et l'élément de châssis supérieur (14).

10. Appareil anatomique (10) pouvant être positionné extérieurement selon la revendication 9, dans lequel la longueur de câble (108) est fabriquée avec des brins de métal tressés.

11. Appareil anatomique (10) pouvant être positionné extérieurement selon la revendication 9, comprenant de plus un contrôleur (114) accessible extérieurement pour allonger ou raccourcir la longueur de câble (108) qui s'étend entre l'élément de bras arrière (102) et l'élément de châssis supérieur (14), régulant ainsi la distance d'extension de membre.

12. Appareil anatomique (10) pouvant être positionné extérieurement selon la revendication 11, dans lequel le contrôleur accessible extérieurement est une tige de vis (116) mise en prise par filetage fixée de manière distale à la longueur de câble, ladite tige (116) étant accessible avec un outil pour une rotation et résultant en l'allongement ou le raccourcissement de la longueur du câble (108).

13. Appareil anatomique (10) pouvant être positionné extérieurement selon la revendication 11, comprenant de plus, une échelle de mesure (126) visible extérieurement pour la longueur du câble (108) de sorte que les longueurs respectives du câble (108) s'étendant entre l'élément de bras arrière (102) et l'élément de châssis supérieur (14) de chaque ensemble pivotant opposé de l'élément d'articulation pivotant (72) peuvent être égalisées.
